# EUROPÄISCHE PATENTSCHRIFT

(11) **EP 3 727 527 B1**
(45) Veröffentlichungstag und Bekanntmachung des Hinweises auf die Patenterteilung: **23.08.2023**
(21) Anmeldenummer: 18833512.9
(22) Anmeldetag: 05.12.2018
(51) Int. Cl.: A61M 5/32

(54) **INJEKTIONSVORRICHTUNG MIT EINER KAPPE ZUR ENTFERNUNG EINER NADELSCHUTZKAPPE VON EINEM PRODUKTBEHÄLTER UND VERFAHREN ZUM VORBEREITEN EINER SOLCHEN INJEKTIONSVORRICHTUNG**
INJECTION DEVICE WITH A CAP FOR REMOVING A NEEDLE PROTECTION CAP FROM A PRODUCT CONTAINER, AND METHOD FOR PROVIDING SUCH AN EJECTION DEVICE
DISPOSITIF D'INJECTION PRÉSENTANT UN CAPUCHON POUR ENLEVER UN CAPUCHON DE PROTECTION D'AIGUILLE D'UN RÉCIPIENT DE PRODUIT ET PROCÉDÉ POUR LA PRÉPARATION D'UN TEL DISPOSITIF D'INJECTION

(30) Priorität: 21.12.2017 CH 15862017
(43) Veröffentlichungstag der Anmeldung: 28.10.2020
(73) Patentinhaber: Ypsomed AG, 3401 Burgdorf (CH)
(72) Erfinder: BURREN, Stefan, 3150 Schwarzenburg (CH); HIRSCHEL, Jürg, 3007 Bern (CH); TSCHIRREN, Markus, 3400 Burgdorf (CH); MELLENBERGER, Andres, 3425 Koppigen (CH)
(74) Vertreter: Meier Obertüfer, Jürg
(86) Internationale Anmeldenummer: PCT/IB2018/059650
(87) Internationale Veröffentlichungsnummer: WO 2019/123073

(56) Entgegenhaltungen:
- EP-B1- 2 255 842
- WO-A1-2013/085454
- WO-A1-2016/205963
- US-A1- 2016 220 764

## Beschreibung

Die Erfindung betrifft eine Injektionsvorrichtung zur Verabreichung eines flüssigen Produkts, insbesondere eines Medikaments. Ferner betrifft die Erfindung ein Verfahren zum Vorbereiten einer solchen Injektionsvorrichtung für die Verabreichung eines Produkts.

Der Begriff "Medikament" umfasst hier jede fliessfähige medizinische Formulierung, welche geeignet ist zur kontrollierten Verabreichung durch ein Mittel wie eine Kanüle oder Hohlnadel hindurch, beispielsweise umfassend eine Flüssigkeit, eine Lösung, ein Gel, oder eine feine Suspension, welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzymen und weitere Substanzen sowohl in fester (suspendierter) oder flüssiger Form aber auch Polysaccaride, Vaccine, DNS oder RNS oder Oglionukleotide, Antikörper oder Teile von Antikörpern sowie geeignete Basis-, Hilfs- und Trägerstoffe.

Aus dem Stand der Technik sind Injektionsvorrichtungen bekannt, in denen eine Fertigspritze angeordnet ist. Die Fertigspritze weist eine Injektionsnadel auf, die unlösbar mit der Fertigspritze verbunden ist und über die ein in der Fertigspritze enthaltenes Medikament ausgegeben werden kann. Um die Injektionsnadel und das Medikament der Fertigspritze steril zu halten, wird die Injektionsnadel von einer an der Fertigspritze befestigten Nadelschutzkappe umschlossen und in Bezug auf die Umgebung steril abgedichtet. Solche Nadelschutzkappen können z.B. als sogenannte soft needle shield (SNS) oder als rigid needle shield (RNS) ausgestaltet sein. Ein soft needle shield (SNS) besteht aus einem elastomeren Teil, welches die Injektionsnadel umgibt. Ein rigid needle shield (RNS) weist mehrere Teile auf, insbesondere ein elastomeres kappenförmiges Teil und ein aus einem festen, d.h. nicht-elastomeren Kunststoff hergestellten hülsenförmiges Teil, welches das elastomere Teil aufnimmt und damit im Wesentlichen unlösbar verbunden ist.

Um ein in der Fertigspritze enthaltenes Medikament injizieren zu können, muss die Nadelschutzkappe von der Fertigspritze entfernt werden. Aus der WO 2010/136076 A1, der US 9,339,610 B2, der WO 2015/144871 A1, WO 2016/205963 A1 und US 2016/0243315 A1 ist es bekannt, dass beim Abziehen eines kappenförmigen Abziehelements, das auch als Kappe bezeichnet wird, am distalen Ende der Injektionsvorrichtung angebracht ist und das distale Ende der Injektionsvorrichtung verschliesst, die an der Fertigspritze angebrachte Nadelschutzkappe mitabgezogen, d. h. beim Entfernen der Kappe von der Fertigspritze entfernt wird. Die Nadelschutzkappe verbleibt dabei in der Kappe. Hierzu weist die Kappe Eingriffsglieder auf, die beim Abziehen der Kappe in Eingriff mit der Nadelschutzkappe gebracht werden. Beim Fortsetzen der Abziehbewegung des Abziehelements nehmen die Eingriffsglieder die Nadelschutzkappe mit, wodurch diese von der Fertigspritze abgezogen wird. Um ein sicheres Abziehen der Nadelschutzkappe durch das Entfernen der Kappe zu gewährleisten, ist es aus dem Stand der Technik bekannt, dass die mit der Kappe verbundenen Eingriffsglieder in Eingriff mit der Nadelschutzkappe gelangen. Ferner ist aus der EP 2255842 B1 und der WO 2013/085454 A1 bekannt, dass durch Drehung der Kappe die Nadelschutzkappe von der Fertigspritze abgezogen werden kann. Dabei ist die Abziehkraft beim Abziehen der Nadelschutzkappe abhängig von dem Weg der Drehbewegung der Kappe, wobei die Abziehkraft bei der Drehbewegung der Kappe kleiner als die Abziehkraft bei einer Axialbewegung der Kappe ist.

Es ist eine Aufgabe der Erfindung, eine Injektionsvorrichtung und ein Verfahren zum Vorbereiten einer solchen Injektionsvorrichtung für die Verabreichung eines Produkts anzugeben, die ein sichereres und/oder einfacheres Entfernen der Nadelschutzkappe von dem Produktbehälter erlaubt.

Die Aufgabe wird mit der Injektionsvorrichtung nach Anspruch 1 und dem Verfahren nach Anspruch 10 gelöst. Vorteilhafte Weiterbildungen ergeben sich aus den abhängigen Ansprüchen, der Beschreibung und den Figuren.

Die Erfindung geht von einer Vorrichtung zur Verabreichung eines Produkts, nämlich von einer Injektionsvorrichtung mit einer Längsachse (L), aus. Die Injektionsvorrichtung kann als so genannter Autoinjektor ausgestaltet sein, der einen Mechanismus aufweist, der ein automatisches Ausschütten des Produkts, wie z. B. durch einen Energiespeicher, insbesondere eine Feder, und optional ein automatisches Einstechen und/oder Zurückziehen der Injektionsnadel bewirkt. Bei einem Autoinjektor wird die Kraft zum Ausschütten des Produkts durch den Energiespeicher, wie z.B. die Feder bereitgestellt. Die Injektionsvorrichtung kann alternativ als manuelle Injektionsvorrichtung ausgestaltet sein, d. h., dass die Kraft für die Ausschüttung des Produkts durch Muskelkraft, wie z. B. durch den Benutzer selbst erfolgt. Die Injektionsvorrichtung - egal ob es sich um einen Autoinjektor oder eine manuelle Injektionsvorrichtung handelt - kann eine Nadelschutzhülse aufweisen, die nach erfolgter Injektion distal über das distale Ende der Injektionsnadel steht oder relativ zu dem Gehäuse in diese Position verschoben wird, um den versehentlichen Zugriff auf die Injektionsnadel zu verhindern und dadurch ein Verletzungsrisiko zu verringern. Bei einem Autoinjektor kann die Nadelschutzhülse beispielsweise auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors als Sichtschutz dient.

Die Injektionsvorrichtung weist einen Produktbehälter mit einer Injektionsnadel auf, wie z. B. eine aus dem Stand der Technik bekannte Fertigspritze oder allgemein Spritze. Der Produktbehälter kann einen z. B. hohlzylindrischen Produktbehälterabschnitt aufweisen, der einen Kolben verschiebbar lagert. Der Kolben kann mit dem Innenumfang des Produktbehälterabschnitts eine Dichtung bilden und so eine sterile Barriere bilden. Der Kolben kann z. B. mittels einer Kolbenstange der Injektionsvorrichtung in die distale Richtung verschoben werden, um über die Injektionsnadel Produkt aus dem Produktbehälter abzugeben. Die Injektionsnadel kann vorzugsweise unlösbar an dem Produktbehälter gebildet sein. Zum Beispiel kann der Produktbehälter einen Halteabschnitt, insbesondere einen Nadelhalteabschnitt, aufweisen, der distal des Produktbehälterabschnitts angeordnet ist und mit der Injektionsnadel unlösbar verbunden ist, und so zum Beispiel einen proximalen Teil der Injektionsnadel umgibt. Die Injektionsnadel kann somit von dem Halteabschnitt in die distale Richtung abragen. Der Halteabschnitt kann beispielsweise einen geringeren Aussendurchmesser als der Produktbehälterabschnitt aufweisen. Der Produktbehälterabschnitt kann sich an seinem distalen Ende zu dem Halteabschnitt hin verjüngen.

Der hierin verwendete Begriff "distal" bezieht sich auf die Richtung, in die die Spitze der Injektionsnadel zeigt. Der hierin verwendete Begriff "proximal" bezieht sich auf die Richtung, die der distalen Richtung entgegengesetzt ist.

An dem Produktbehälter, beispielsweise an dem Halteabschnitt, ist eine Nadelschutzkappe, wie z. B. ein aus dem Stand der Technik bekanntes soft needle shield (SNS) oder rigid needle shield (RNS), befestigt, insbesondere lösbar befestigt. Die Nadelschutzkappe kann z. B. reib- oder formschlüssig oder kombiniert reib- und formschlüssig auf dem Halteabschnitt befestigt sein. Die Nadelschutzkappe umschliesst die Injektionsnadel und dichtet sie in Bezug auf die Umgebung steril ab. Ein soft needle shield (SNS) umfasst oder besteht aus einem Elastomer, beispielsweise auf Gummi- oder Kautschukbasis gebildeten Teil, welches die Nadel umgibt. Das soft needle shield (SNS) weist an seinem Aussenumfang eine weiche, wie z. B. aus einem gummi- oder kautschukartigen Material gebildete Oberfläche auf. Ein rigid needle shield (RNS) weist zumeist mehrere Teile auf, insbesondere ein elastomeres kappenförmiges inneres Teil und ein aus einem steiferen, d. h. nicht-elastomeren Kunststoff hergestelltes hülsenförmiges oder kappenförmiges äusseres Teil, welches das elastomere Teil aufnimmt und damit im Wesentlichen unlösbar verbunden ist. Das äussere hülsen- oder kappenförmige Teil umgibt das innere kappenförmige Teil und ist mit der inneren Kappe beispielsweise unlösbar verbunden, so dass die äussere und innere Kappe eine Einheit bilden. Das äussere Teil kann aus einem härteren Kunststoff gebildet als das innere Teil sein. Das äussere Teil kann beispielsweise aus Polyethylen, Polystyrol, Polypropylen oder einem anderen geeigneten Kunststoff sein. Das innere Teil kann beispielsweise aus Gummi oder Kautschuk oder einem anderen geeigneten Material gebildet sein.

An dem distalen Ende der Injektionsvorrichtung oder eines Gehäuses, wie z. B. eines Aufnahmegehäuses der Injektionsvorrichtung kann eine Kappe, die auch als Verschlusskappe oder Abziehkappe bezeichnet werden oder ausgestaltet sein kann, befestigt sein und das distale Ende des Gehäuses oder des Aufnahmegehäuses verschliessen. Die Injektionsvorrichtung kann ein Gehäuse, wie z.B. ein Aufnahmegehäuse der Injektionsvorrichtung zur Aufnahme des Produktbehälters umfassen, wobei der Produktbehälter eine fest verbundene Injektionsnadel aufweist und wobei an dem Produktbehälter die Nadelschutzkappe lösbar angeordnet ist. Die Nadelschutzkappe umschliesst die Injektionsnadel und dichtet die Injektionsnadel gegenüber der Umgebung steril ab. Die Kappe kann z. B. mit dem Gehäuse oder Aufnahmegehäuse reib- und/oder formschlüssig verbunden sein, wie z. B. verschnappt sein. Die Kappe kann z. B. während des Entfernens von der Injektionsvorrichtung oder dem Gehäuse mit einer kombinierten Axial-Dreh-Bewegung und einer Axial-Bewegung von der Injektionsvorrichtung, wie z. B. dem Gehäuse oder Aufnahmegehäuse, abnehmbar sein.

Die Injektionsvorrichtung kann ferner einen Produktbehälterhalter umfassen, welcher mit dem Gehäuse der Injektionsvorrichtung fest, insbesondere axial- und drehfest verbunden ist. Der Produktbehälterhalter kann zur Aufnahme des Produktbehälters dienen, wobei in dem Produktbehälterhalter der Produktbehälter fest, insbesondere axial- und vorzugsweise drehfest gehalten werden kann. Alternativ können das Gehäuse und der Produktbehälterhalter einteilig ausgebildet sein. Alternativ kann der Produktbehälterhalter relativ zu dem Gehäuse axial bewegbar und/oder drehbar angeordnet sein.

Die Kappe, welche lösbar an dem distalen Ende des Gehäuses der Injektionsvorrichtung vorgesehen ist, umfasst ein oder mehrere Eingriffselemente, um beim Entfernen der Kappe von der Injektionsvorrichtung das Entfernen der Nadelschutzkappe von dem Produktbehälter zu bewirken. Die Kappe, welche mit dem Eingriffselement gekoppelt ist, kann über das Eingriffselement derart mit der Nadelschutzkappe verbindbar sein, dass das Entfernen der Kappe von der Injektionsvorrichtung das Entfernen der Nadelschutzkappe von dem Produktbehälter bewirkt. Insbesondere kann zumindest ein Teil der Bewegung oder die gesamte Bewegung der Kappe in die distale Richtung auf das Eingriffselement übertragen werden, d. h., dass das Eingriffselement von der Kappe mitgenommen wird, so dass das Eingriffselement die Nadelschutzkappe von dem Produktbehälter, insbesondere dem Halteabschnitt, abzieht. Vorzugsweise ist das Eingriffselement relativ zu dem Gehäuse drehfest angeordnet.

In einer Ausführungsform kann das Eingriffselement derart verformbar sein, dass das Eingriffselement von einer beabstandeten Position, in welcher das Eingriffselement von der Nadelschutzkappe radial beabstandet ist, in eine Eingriffsposition, in welcher das Eingriffselement in Eingriff mit der Nadelschutzkappe ist, bringbar ist, wobei das Eingriffselement beim Entfernen der Kappe verformt wird. Das Eingriffselement kann z. B. im Auslieferungszustand der Injektionsvorrichtung in Bezug auf die Nadelschutzkappe in der beabstandeten Position sein. In der Eingriffsposition ist das Eingriffselement in Bezug auf die Nadelschutzkappe derart angeordnet, dass eine Bewegung der Kappe in die distale Richtung eine Mitnahme der Nadelschutzkappe bewirkt und somit die Nadelschutzkappe von dem Produktbehälter entfernt wird. In der Eingriffsposition des Eingriffselements greift das Eingriffselement an oder in die Nadelschutzkappe. Das Eingriffselement kann an oder in eine Mantelfläche oder an oder in eine Kante oder an oder in eine distalen Stirnfläche oder an oder in eine proximale Stirnfläche der Nadelschutzkappe greifen. Das Eingriffselement kann einen Haken oder mehrere Haken umfassen. Besonders bevorzugt kann das Eingriffselement zumindest teilweise hakenförmig ausgebildet sein. Besonders bevorzugt kann das Eingriffselement ferner hülsenförmig oder zylinderförmig ausgebildet sein. Das hakenförmige Eingriffselement kann einen kurzen und einen langen Schenkel aufweisen. Vorzugsweise kann der lange Schenkel verformbar ausgebildet sein. Ferner kann der kurze Schenkel zahn- oder dreieckförmig oder spitzwinklig ausgebildet sein. Alternativ kann das Eingriffselement eine andere Ausgestaltung aufweisen, wobei in der beabstandeten Position des Eingriffselements das Eingriffselement von der Nadelschutzkappe radial beabstandet ist und in der Eingriffsposition des Eingriffselements das Eingriffselement in Eingriff mit der Nadelschutzkappe ist, wobei das Eingriffselement beim Entfernen der Kappe verformt wird.

In der beabstandeten Position des Eingriffselements kann das Eingriffselement unverformt, verformt oder radial nach aussen verformt sein. In der Eingriffsposition des Eingriffselements kann das Eingriffselement unverformt, verformt oder radial nach innen verformt sein. Das Eingriffselement kann vorzugsweise plastisch und/oder elastisch verformbar sein.

Als eine plastische Verformung wird eine bleibende Verformung bezeichnet. Die Verformung eines Materials ist plastisch, wenn das Material nicht wieder von allein seine ursprüngliche Form annimmt. Nach Einwirkung von einer Kraft oder Belastung auf das Material behält das Material seine Form bei. Als eine elastische Verformung wird eine reversible Verformung bezeichnet. Dabei nimmt ein Material nach Einwirkung einer Kraft oder einer Belastung auf das Material wieder seine ursprüngliche Form an.

Das Eingriffselement ist vorzugsweise aus Metall, insbesondere aus Stahl, besonders bevorzugt aus rostfreiem Stahl oder Federstahl gebildet. Das Eingriffselement ist aus einem Material gebildet, welches eine Biegefestigkeit aufweist, welche eine plastische und/oder elastische Verformung zulässt. Besonders bevorzugt ist das Eingriffselement derart ausgebildet, dass es in der beabstandeten Position des Eingriffselements plastisch und/oder elastisch verformt und in der Eingriffsposition plastisch und/oder elastisch unverformt ausgebildet ist oder, dass es in der beabstandeten Position des Eingriffselements plastisch und/oder elastisch unverformt und in der Eingriffsposition plastisch und/oder elastisch verformt ausgebildet ist.

Ferner kann an dem Gehäuse oder an einem Gehäuse fest verbundenen Teil ein oder mehrere Sperrelemente vorgesehen sein, wobei in der Eingriffsposition das oder die Sperrelemente das Eingriffselement in Eingriff mit der Nadelschutzkappe hält oder bringt. Das Sperrelement kann eine erste und/oder eine zweite schräge Fläche, insbesondere eine erste und/oder eine zweite nach innen ragende schräge Fläche, umfassen. Die erste und/oder die zweite schräge Fläche des Sperrelements können eine Neigung aufweisen. Die erste und die zweite schräge Fläche können zueinander geneigt sein. Das Eingriffselement der Kappe ist derart mit dem Sperrelement des Gehäuses gekoppelt, dass während dem Entfernen der Kappe von der Injektionsvorrichtung das Eingriffselement relativ zu der Nadelschutzkappe bewegbar ist oder bewegt wird und bei dieser Bewegung, insbesondere einer Axialbewegung, mittels dem Sperrelement des Gehäuses derart verformbar ist oder verformt wird, dass das Eingriffselement, insbesondere der kurze Schenkel des Eingriffselements in Eingriff mit der Nadelschutzkappe gelangbar ist oder gelangt. In der Eingriffsposition des Eingriffselements ist das Eingriffselement mit der Nadelschutzkappe axialfest verbunden, wobei die Nadelschutzkappe von dem Eingriffselement der Kappe bei der Fortführung der kombinierten Axial-Drehbewegung der Kappe mitgenommen wird. Mit anderen Worten umfasst der Hub, den die Kappe beim Entfernen von der Injektionsvorrichtung relativ dem Gehäuse entlang der Längsachse (L) in die distale Richtung ausführt, einen ersten Teilhub, während dem die Kappe relativ zu der Nadelschutzkappe bewegbar ist oder bewegt wird, und einen zweiten Teilhub, während dem die Nadelschutzkappe die Bewegung der Kappe mitmacht oder von der Kappe mitgenommen wird.

In einer alternativen Ausführungsform kann ein oder mehrere Eingriffselemente vorgesehen sein, welche elastisch und/oder plastisch verformbar ausgebildet sind, wobei das oder die mehreren Eingriffselemente immer und/oder bereits beim Montieren einer Injektionsvorrichtung für die Verabreichung eines Produkts in einem Kontakt mit der Nadelschutzkappe sind. Dieses Montageverfahren umfasst ferner das Verschieben oder Einsetzen eines Produktbehälters mit der lösbar verbundenen Nadelschutzkappe in ein Gehäuse entlang einer Längsachse (L) in eine distale Richtung, wobei das Gehäuse an einem distalen Ende eine Kappe aufweist. Eine Mantelaussenfläche der Nadelschutzkappe gleitet dabei axial über das oder die Eingriffselemente, insbesondere den oder die kurzen Schenkel des Eingriffselements. In der Position, in welcher der Produktbehälter in dem Gehäuse eingesetzt ist, ist das oder die Eingriffselemente der Kappe in einer Eingriffsposition.

In einer alternativen Ausführungsform kann die Kappe mit einem oder mit mehreren Eingriffselementen relativ zu einer Nadelschutzkappe in die proximale Richtung bewegt werden, um die Kappe auf ein Produktbehälter aufgenommenes Gehäuse aufzusetzen.

Die Mantelfläche der Nadelschutzkappe kann eine oder mehrere Öffnungen oder ein oder mehrere Befestigungsmittel aufweisen, in welche das Eingriffselement in der Eingriffsposition des Eingriffselements eingreifen oder einbohren kann. Alternativ weist die Nadelschutzkappe keine Öffnung oder kein Befestigungsmittel auf, wobei das Eingriffselement in der Eingriffsposition des Eingriffselements in die Mantelfläche der Nadelschutzkappe eingreifen oder einbohren kann.

Ferner kann die Kappe eine Drehhülse aufweisen, wobei die Drehhülse das Eingriffselement zumindest teilweise aufgenommen hat, und welche relativ zu dem Gehäuse und relativ zu dem Eingriffselement um die Längsachse (L) drehbar ist, und welche relativ zu dem Eingriffselement axialfest angeordnet ist. Vorzugsweise sind das Eingriffselement und die Drehhülse zueinander konzentrisch angeordnet. Die Drehhülse und das Gehäuse weisen jeweils eine wellenförmige oder kurvenförmige Führungskulisse auf, welche derart ausgebildet sind und derart zusammenwirken, dass die Drehhülse relativ zu dem Gehäuse um die Längsachse (L) drehbar ist und die Drehhülse und das Eingriffselement relativ zu dem Gehäuse axial in die distale Richtung bewegbar ist. Das Eingriffselement ist relativ zu dem Gehäuse drehfest angeordnet. Die wellenförmige oder kurvenförmige Führungskulisse ist in Gleitkontakt mit der wellenförmige oder kurvenförmige Führungskulisse des Gehäuses. Besonders bevorzugt weist das distale Ende der Drehhülse die wellenförmige oder kurvenförmige Führungskulisse auf, welche in Gleitkontakt mit der wellenförmigen oder kurvenförmigen Führungskulisse des Gehäuses ist, wobei die Führungskulisse des Gehäuses als ein an einer Mantelaussenfläche des Gehäuses umlaufenden Vorsprung oder Steg ausgebildet ist. Besonders bevorzugt sind die wellenförmigen oder kurvenförmigen Führungskulissen der Drehhülse und des Gehäuses derart ausgebildet und wirken derart zusammen, dass nach einer relativen Drehung der Drehhülse um etwa 90° um die Längsachse (L), die Drehhülse relativ zu dem Gehäuse an einer distalsten Position angeordnet ist. Das heisst, dass sich bei einer relativen Drehung der Drehhülse um etwa 90° um die Längsachse (L) die Drehhülse relativ zu dem Gehäuse um einen maximalen axialen Weg in distaler Richtung bewegt. Bei Drehung der Drehhülse um die Längsachse (L) findet gleichzeitig eine axiale Bewegung des Eingriffselements in die distale Richtung statt, wobei das Eingriffselement in Eingriff mit der Nadelschutzkappe ist oder gebracht wird.

Die Drehhülse kann vorzugsweise aus Kunststoff gebildet sein. Alternativ kann die Drehhülse aus Metall gebildet sein. Die Drehhülse kann z.B. mit dem Gehäuse der Injektionsvorrichtung reib- und/oder formschlüssig verbunden sein, wie z.B. verschnappt sein. Dazu kann z.B. an der Drehhülse ein Eingriffsglied vorgesehen sein, welches in ein an dem Gehäuse angebrachtes Gegeneingriffsglied lösbar in Eingriff sein kann. Die Drehhülse ist mit einer kombinierten Axial-Dreh-Bewegung und einer Axial-Bewegung von der Injektionsvorrichtung, insbesondere von dem Gehäuse abnehmbar. Insbesondere ist die Drehhülse in einem ersten Schritt durch eine kombinierte Axial-Dreh-Bewegung und in einem zweiten Schritt durch ein axiales Entfernen von der Injektionsrichtung, insbesondere von dem Gehäuse abnehmbar.

Die Drehhülse weist ein Rückdrehsicherungselement und das Eingriffselement oder das Gehäuse ein Rückdrehsicherungsgegenelement auf, um eine Drehung der Drehhülse um die Längsachse (L) in eine Richtung zu ermöglichen und in Gegenrichtung zu sperren. Der Rückdrehmechanismus der Injektionsvorrichtung dient dazu, dass der Benutzer die Drehhülse nur in eine Drehrichtung relativ zu dem Eingriffselement oder relativ zu dem Gehäuse drehen kann. Eine Drehung der Drehhülse um die Längsachse (L) ist in eine Richtung möglich und in die Gegenrichtung blockiert.

Besonders bevorzugt kann das Rückdrehsicherungselement der Drehhülse und/oder das Rückdrehsicherungsgegenelement des Eingriffelements oder des Gehäuses eine Verzahnung oder Verrastung aufweisen oder als Verzahnung oder Verrastung ausgebildet sein. Dazu kann die Drehhülse ein oder mehrere Zähne aufweisen, welche mit einem oder mit mehreren Zähnen des Eingriffselements oder des Gehäuses derart zusammenwirken, dass eine Drehung der Drehhülse um die Längsachse (L) in eine Richtung möglich ist und in Gegenrichtung gesperrt ist. Die korrespondierende Verzahnung oder Verrastung umfassen entsprechende Gleitflächen und Anschlagsflächen auf, um eine Drehung der Drehhülse um die Längsachse (L) in eine Richtung zu ermöglichen und in Gegenrichtung zu sperren.

In einer alternativen Ausführungsform kann das Rückdrehsicherungselement der Drehhülse und/oder das Rückdrehsicherungsgegenelement des Eingriffselements oder des Gehäuses ein Federarm aufweisen oder als Federarm ausgebildet sein. Ferner kann das Rückdrehsicherungselement der Drehhülse und/oder das Rückdrehsicherungsgegenelement des Eingriffselements oder des Gehäuses einen Vorsprung, Absatz, Vertiefung, Rille oder Aussparung aufweisen, welche um die Längsachse (L) in eine Richtung möglich ist und in Gegenrichtung gesperrt ist.

Die Kappe kann ferner eine hülsenförmige oder zylinderförmige Entfernerhülse aufweisen, welche axialfest und drehfest mit dem Eingriffselement verbunden ist und wobei die Drehhülse relativ zu der Entfernerhülse drehbar angeordnet ist. Alternativ können die Entfernerhülse und das Eingriffselement einstückig ausgebildet sein. Alternativ kann das Eingriffselement die Entfernerhülse umfassen. Besonders bevorzugt können die Drehhülse und die Entfernerhülse in einem Führungseingriff sein, derart, dass die Drehhülse relativ zu der Entfernerhülse um die Längsachse (L) drehbar ist. Des Weiteren kann die Entfernerhülse drehfest mit dem Gehäuse verbunden sein. Die Entfernerhülse ist vorzugsweise aus Kunststoff gefertigt. Alternativ kann die Entfernerhülse aus dem gleichen Material wie das Eingriffselement gefertigt sein.

In einer alternativen Ausführungsform kann keine Entfernerhülse vorgesehen sein. Die Drehhülse und das Eingriffselement können in einem Führungseingriff sein, derart dass die Drehhülse relativ zu dem Eingriffselement um die Längsachse (L) drehbar ist. Des Weiteren kann das Eingriffselement drehfest mit dem Gehäuse verbunden sein.

In einer alternativen Ausführungsform kann die Drehhülse um einen Drehwinkel um die Längsachse (L) in eine Richtung gedreht werden, wobei nach Drehung der Drehhülse um einen bestimmten Drehwinkel eine Drehung der Drehhülse um die Längsachse (L) in Gegenrichtung blockiert ist. Dazu kann die Drehhülse eine Rückdrehnocke aufweisen und die Entfernerhülse einen Rückdrehvorsprung umfassen, welche derart zusammen wirken, dass bei Drehung der Drehhülse um die Längsachse (L) in eine Richtung die Rückdrehnocke über den Rückdrehvorsprung gleiten kann, aber bei Drehung der Drehhülse um die Längsachse (L) in Gegenrichtung die Rückdrehnocke in Anschlagkontakt mit dem Rückdrehvorsprung gelangt und die Drehung in Gegenrichtung blockiert wird.

In einer alternativen Ausführungsform kann die Drehhülse in eine erste und/oder in eine zweite Drehrichtung um die Längsachse (L) gedreht werden, wobei nach Drehung der Drehhülse um einen bestimmten Drehwinkel um die Längsachse (L) in die erste und/oder zweite Drehrichtung eine Drehung der Drehhülse um die Längsachse (L) in Gegenrichtung der ersten und/oder der zweiten Drehrichtung blockiert ist. Dazu kann die Drehhülse eine Rückdrehnocke aufweisen und die Entfernerhülse ein Paar Rückdrehvorsprünge umfassen, welche derart zusammenwirken, dass bei Drehung der Drehhülse um die Längsachse (L) in eine erste und/oder zweite Richtung die Rückdrehnocke über den Rückdrehvorsprung gleiten kann, aber bei Drehung der Drehhülse um die Längsachse (L) in Gegenrichtung der ersten und/oder zweiten Richtung die Rückdrehnocke in Anschlagkontakt mit einem der Rückdrehvorsprünge gelangt und die Drehung in Gegenrichtung blockiert wird.

Alternative Ausgestaltungen des Rückdrehsicherungselements der Drehhülse und/oder des Rückdrehsicherungsgegenelements des Eingriffselements oder des Gehäuses können vorgesehen sein, soweit dass eine Drehung der Drehhülse um die Längsachse (L) in eine Richtung möglich ist und in Gegenrichtung gesperrt wird.

Ferner können die Drehhülse, das Eingriffselement und/oder das Gehäuse eine visuelle Markierung, insbesondere in Form eines Zeichens, besonders bevorzugt in Form eines Pfeils aufweisen, um anzuzeigen in welche Richtung eine Drehung der Drehhülse um die Längsachse (L) möglich ist. Diese Markierung dient der einfacheren Bedienung der Injektionsvorrichtung. In alternativen Ausführungsformen kann zusätzlich oder alternativ eine akustische und/oder taktile Markierung vorgesehen sein.

Bevorzugt kann ferner eine Nadelschutzhülse vorgesehen sein, wobei die Kappe über die Nadelschutzhülse mit dem Gehäuse lösbar verbunden ist. Die Nadelschutzhülse ist vorzugsweise relativ zu dem Gehäuse drehfest angeordnet. Die Nadelschutzhülse dient dazu, vor oder nach erfolgter Injektion distal über das distale Ende der Injektionsnadel zu stehen. Die Nadelschutzhülse ist teilweise von dem Gehäuse aufgenommen, wobei an einem distalen Ende der Nadelschutzhülse die Kappe aufgesetzt werden kann. Die Kappe kann z. B. mit der Nadelschutzhülse reib- und/oder formschlüssig verbunden sein, wie z. B. verschnappt werden. Besonders bevorzugt werden das Eingriffselement und/oder die Entfernerhülse der Kappe mit der Nadelschutzhülse lösbar verbunden. Des Weiteren können das Eingriffselement und/oder die Entfernerhülse drehfest mit der Nadelschutzhülse verbunden sein. Alternativ kann die Kappe mit dem Gehäuse lösbar verbunden sein, wobei die Injektionsvorrichtung eine oder keine Nadelschutzhülse umfassen kann.

Ergänzend wird auf die Merkmale, die im Zusammenhang mit der hierin beschriebenen Vorrichtung offenbart werden, verwiesen, die auch die Vorrichtung für das Verfahren vorteilhaft weiterbilden.

Die Erfindung wird anhand von mehreren Figuren beschrieben. Die dabei offenbarten Merkmale bilden die Erfindung einzeln und in jeglicher Merkmalskombination vorteilhaft weiter. Es zeigen:
Figur 1a eine Explosionsansicht einer Ausführungsform einer erfindungsgemässen Injektionsvorrichtung.
Figur 1b eine Perspektivenansicht einer Drehhülse (3) der Injektionsvorrichtung gemäss Figur 1a.
Figur 1c eine Perspektivenansicht einer Entfernerhülse (5) der Injektionsvorrichtung gemäss Figur 1a.
Figur 2 eine Längsschnittansicht der Injektionsvorrichtung gemäss Figur 1, wobei eine Kappe (2) lösbar an einem distalen Ende eines Gehäuses (1) vorgesehen ist und wobei ein Produktbehälter mit einer Nadelschutzkappe nicht ersichtlich ist.
Figur 3a eine Aussenansicht der Injektionsvorrichtung gemäss Figur 2.
Figur 3b eine Aussenansicht der Injektionsvorrichtung gemäss Figur 3a, wobei eine Drehhülse (3) um die Längsachse (L) um etwa 90° gedreht ist.

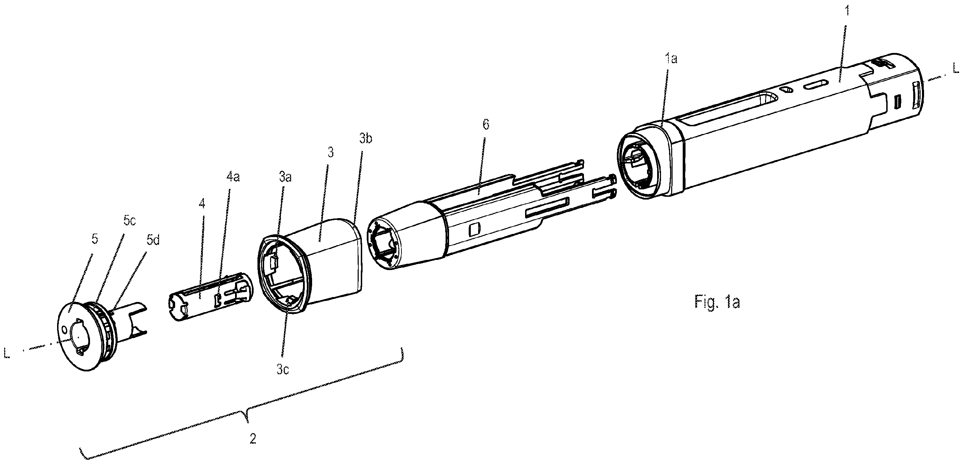

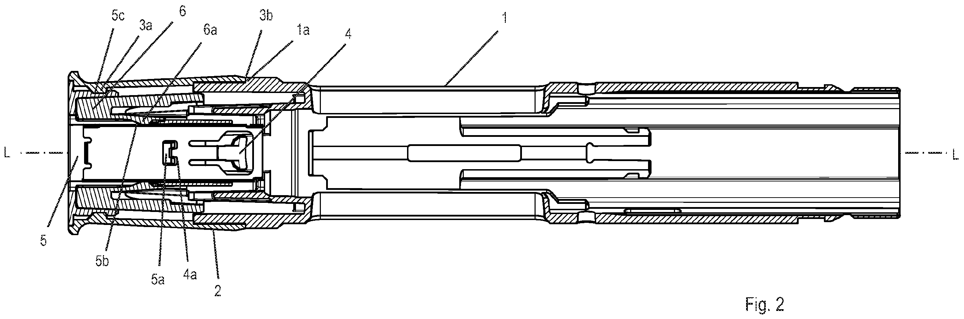

In der Figur 1a ist eine Explosionsansicht einer Ausführungsform einer erfindungsgemässen Injektionsvorrichtung ersichtlich. Die Injektionsvorrichtung mit einer Längsachse (L) umfasst ein Gehäuse (1). Das Gehäuse (1) kann als hülsenförmiges, insbesondere zylindrisches Aufnahmegehäuse (1) mit einem distalen und einem proximalen Ende ausgebildet sein. An dem distalen Ende des Gehäuses (1) kann die Kappe (2) lösbar vorgesehen sein. Das Gehäuse (1) dient zur Aufnahme eines Produktbehälters (nicht gezeigt), wobei der Produktbehälter (nicht gezeigt) eine fest verbundene Injektionsnadel aufweist, wobei an dem Produktbehälter (nicht gezeigt) eine Nadelschutzkappe lösbar angeordnet ist, welche die Injektionsnadel umschliesst und gegenüber der Umgebung steril abdichtet. Die Injektionsvorrichtung umfasst ferner eine Nadelschutzhülse (6). Die Nadelschutzhülse (6) kann relativ zu dem Gehäuse (1) der Injektionsvorrichtung zum Auslösen einer Produktausschüttung in die proximale Richtung verschiebbar sein. Die Nadelschutzhülse (6) ist vorzugsweise drehfest mit dem Gehäuse (1) verbunden. Nach erfolgter Produktausschüttung kann die Nadelschutzhülse (6) relativ zu dem Gehäuse (1) in die distale Richtung verschiebbar sein, um die Spitze der Injektionsnadel abzudecken, um eine Verletzungsgefahr zu verringern. Die Kappe (2) umfasst eine Drehhülse (3), ein oder mehrere Eingriffselemente (4) und eine Entfernerhülse (5). Das Eingriffselement (4) dient beim Entfernen der Kappe (2) von der Injektionsvorrichtung dazu, das Entfernen der Nadelschutzkappe von dem Produktbehälter (nicht gezeigt) zu bewirken. Dazu ist das Eingriffselement (4) hakenförmig ausgebildet oder weist einen oder mehrere Haken auf. Der Haken ist derart ausgebildet, dass der Haken in oder an die Nadelschutzkappe greifen kann, um in einer Eingriffsposition mit der Nadelschutzkappe zu sein oder zu gelangen. Das Eingriffselement (4) ist vorzugsweise aus Metall, insbesondere aus Stahl, besonders bevorzugt aus rostfreiem Stahl oder Federstahl gebildet. Das Eingriffselement (4) ist axialfest und drehfest mit der Entfernerhülse (5) verbunden. Dazu weist das Eingriffselement (4) eine Aussparung (4a) und die Entfernerhülse (5) einen Vorsprung (5a) auf. Der Vorsprung (5a) der Entfernerhülse (5) ist in die Aussparung (4a) des Eingriffselements (4) fest bzw. unlösbar eingeschnappt. Die Entfernerhülse (5) ist hülsenförmig oder zylinderförmig ausgebildet. Die Entfernerhülse (5) ist vorzugsweise aus Kunststoff gebildet. Alternativ können das Eingriffselement (4) und die Entfernerhülse (5) einstückig ausgebildet sein und vorzugsweise aus Metall, insbesondere aus Stahl, besonders bevorzugt aus rostfreiem Stahl oder Federstahl gebildet sein. Die Kappe (2) ist vorzugsweise über eine Schnappverbindung zwischen der Entfernerhülse (5) und der Nadelschutzhülse( 6) lösbar mit der Nadelschutzhülse (6) und/oder dem Gehäuse (1) verbunden. Dazu weist die Nadelschutzhülse (6) eine Abragung (Figur2; 6a) auf, welche in eine durchgängige oder undurchgängige Öffnung (Figur 2; 5b) der Entfernerhülse (5) lösbar schnappen oder ragen kann. Die Kappe (2) ist somit über die die Nadelschutzhülse (6) an dem distalen Ende des Gehäuses (1) lösbar vorgesehen. In einer alternativen Ausführungsform kann die Kappe (2) direkt an dem distalen Ende des Gehäuses (1) lösbar vorgesehen sein. Die Drehhülse (3) nimmt zumindest teilweise das Eingriffselement (4) auf. Vorzugsweise sind die Drehhülse (3) und das Eingriffselement (4) zueinander konzentrisch angeordnet. Die Drehhülse (3) ist relativ zu dem Gehäuse (1) und relativ zu dem Eingriffselement (4) um die Längsachse (L) drehbar angeordnet. Besonders bevorzugt sind die Drehhülse (3) und die Entfernerhülse (5) in einem Führungseingriff, derart, dass die Drehhülse (3) relativ zu der Entfernerhülse (5) um die Längsachse (L) drehbar ist. Dazu weist die Drehhülse (3) an einer Mantelinnenfläche einen Nocken (3a) auf, welcher in Eingriff mit einer Ringnut (5c) der Entfernerhülse (5) ist. Die Ringnut (5c) ist an einer Mantelaussenfläche der Entfernerhülse (5) vorgesehen. Die Entfernerhülse (5) ist drehfest mit der Nadelschutzhülse (6) und/oder dem Gehäuse (1) verbunden. Dazu weist die Entfernerhülse (5) eine Längsnut (5d) auf, welcher in Eingriff mit einem Längssteg (nicht gezeigt) der Nadelschutzhülse (6) ist. Alternativ kann die Entfernerhülse (5) drehfest über eine Längssteg/Längsnut-Verbindung mit dem Gehäuse (1) verbunden sein. Ferner weisen die Drehhülse (3) und das Gehäuse (1) jeweils eine wellenförmige oder kurvenförmige Führungskulisse (3b; 1a) auf, welche derart ausgebildet sind und derart zusammenwirken, dass die Drehhülse (3) relativ zu dem Gehäuse (1) um die Längsachse (L) drehbar ist und die Drehhülse (3) und das Eingriffselement relativ zu dem Gehäuse (1) axial in die distale Richtung bewegbar ist. Dazu umfasst die Drehhülse (3) an einem proximalen Ende eine wellenförmige oder kurvenförmige Führungskulisse (3b) und das Gehäuse (1) weist an einem distalen Ende eine korrespondierende wellenförmige oder kurvenförmige Führungskulisse (1a) auf. Besonders bevorzugt weist das Gehäuse (1) an einer Mantelaussenfläche des Gehäuses (1) einen umlaufenden Vorsprung oder Steg auf, welcher die korrespondierende wellenförmige oder kurvenförmige Führungskulisse (1a) bildet. Die wellenförmigen oder kurvenförmigen Führungskulissen der Drehhülse (3b) und des Gehäuses (1a) sind derart ausgebildet und wirken derart zusammen, dass nach einer relativen Drehung der Drehhülse (3) um etwa 90° um die Längsachse (L), sich die Drehhülse (3) relativ zu dem Gehäuse (1) an einer distalen Position befindet. Bei einer relativen Drehung der Drehhülse um etwa 90° um die Längsachse (L), wird die Drehhülse (3) relativ zu dem Gehäuse um einen axialen Weg in distaler Richtung bewegt, wobei bei Drehung der Drehhülse (3) um die Längsachse (L) gleichzeitig eine axiale Bewegung des Eingriffselements (4) in die distale Richtung stattfindet.

Ferner weist die Injektionsvorrichtung ein Rückdrehsicherungsmechanismus, insbesondere in Form eines Rückdrehsicherungselementes und eines Rückdrehsicherungsgegenelement auf, um eine Drehung der Drehhülse (3) um die Längsachse (L) in eine Richtung zu ermöglichen und in Gegenrichtung zu sperren. In einer Ausführungsform umfasst die Drehhülse (3) ein Rückdrehsicherungselement und die Entfernerhülse (5) weist ein Rückdrehsicherungsgegenelement auf, um eine Drehung der Drehhülse (3) um die Längsachse (L) in eine Richtung zu ermöglichen und in Gegenrichtung zu sperren. Der Rückdrehmechanismus der Injektionsvorrichtung dient dazu, dass der Benutzer die Drehhülse (3) nur in eine Drehrichtung um die Längsachse (L) relativ zu dem Eingriffselement (4) oder relativ zu dem Gehäuse (1) drehen kann. Die Drehhülse (3) kann eine visuelle Markung, insbesondere in Form eines Zeichens, beispielsweise eines Pfeils aufweisen, um anzuzeigen, in welche Richtung eine Drehung der Drehhülse (3) um die Längsachse (L) möglich ist.

In einem Ausführungsbeispiel kann die Drehhülse (3) einen Federarm aufweisen, welcher elastisch spannbar, insbesondere radial nach aussen elastisch spannbar ist. Der Federarm kann vorzugsweise an einer Mantelinnenfläche der Drehhülse (3) angeordnet sein. Die Entfernerhülse (5) kann eine oder besonders bevorzugt mehrere Rillen, insbesondere Längsrillen aufweisen. Die Rille, besonders bevorzugt die Rillen, insbesondere die Längsrillen sind an einer Mantelfläche in Umfangsrichtung, insbesondere gleichmässig verteilt angeordnet. Der Federarm der Drehhülse (3) und die Längsrillen der Entfernerhülse (5) sind derart ausgebildet und wirken derart zusammen, dass eine Drehung der Drehhülse (3) um die Längsachse (L) in eine Richtung möglich ist und in Gegenrichtung gesperrt ist.

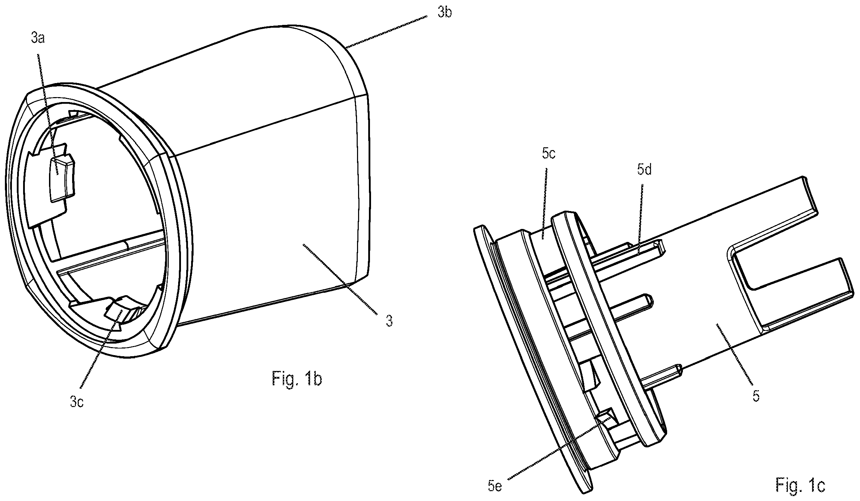

In einem anderen Ausführungsbeispiel kann die Drehhülse (3) eine Rückdrehnocke (3c) aufweisen, wie in der Figur 1b dargestellt ist. Besonderes bevorzugt umfasst die Drehhülse (3) mehrere Rückdrehnocken (3). Ferner kann die Entfernerhülse (5) einen oder mehrere Rückdrehvorsprünge (5e) aufweisen. Der Rückdrehvorsprung (5e) der Entfernerhülse (5) kann zahnförmig ausgebildet sein. Der Rückdrehvorsprung (5e) der Entfernerhülse (5) ist zägezahnförmig ausgebildet. Die schräge Fläche des Rückdrehvorsprungs (5e) und die Rückdrehnocke (3c) der Drehhülse sind derart ausgebildet, dass der Rückdrehvorsprung (5e) bei einer Drehung der Drehhülse (3) um die Längsachse (L), insbesondere bei einer Drehung der Drehhülse (3) um die Längsachse (L) in eine erste Drehrichtung die Rückdrehnocke (3c) über die schräge Fläche des Rückdrehvorsprungs (5e) gleiten kann. Ferner sind die steile Fläche des Rückdrehvorsprungs (5e) und die Rückdrehnocke (3c) der Drehhülse derart ausgebildet, dass der Rückdrehvorsprung (5e) eine Drehung der Drehhülse (3) um die Längsachse (L), insbesondere eine Drehung der Drehhülse um die Längsachse (L) in eine der ersten Drehrichtung entgegengesetzten Drehrichtung blockiert.

In einem besonders bevorzugten Ausführungsbeispiel, wie in der Figur 1c dargestellt, kann die Entfernerhülse (5) ein oder mehrere Paare Rückdrehvorsprünge (5e) aufweisen. Ein Paar von Rückdrehvorsprüngen (5e) umfasst zwei Rückdrehvorsprünge (5e), welche in Umfangsrichtung zueinander entgegengesetzt angeordnet sind. Diese Anordnung dient dazu, dass die Drehhülse (3) um die Längsachse (L) sowohl in eine erste und/oder in eine zweite Drehrichtung gedreht werden kann, wobei nach dem Drehen in die erste und/oder in die zweite Drehrichtung eine Drehung der Drehhülse (3) um die Längsachse (L) in eine der ersten Drehrichtung entgegengesetzten Drehrichtung und/oder eine Drehung der Drehhülse (3) um die Längsachse (L) in eine der zweiten Drehrichtung entgegengesetzten Drehrichtung blockiert wird.

In der Figur 2 ist eine Längsschnittansicht der Injektionsvorrichtung gemäss Figur 1 ersichtlich, wobei eine Kappe (2) lösbar an dem distalen Ende des Gehäuses (1) vorgesehen ist und wobei der Produktbehälter (nicht gezeigt) mit der Nadelschutzkappe nicht ersichtlich ist. In der Figur 2 ist die Injektionsvorrichtung in einem Auslieferungszustand dargestellt, wobei die Kappe (2) an dem distalen Ende aufgesetzt ist. Die Kappe (2) ist an dem distalen Endes des Gehäuses (1), insbesondere über Abragung (6a) der Nadelschutzhülse (6) und über die Öffnung (5b) der Entfernerhülse (5) an dem distalen Ende einer Nadelschutzhülse (6) lösbar vorgesehen. Die Drehhülse (3) kann ferner über eine reib- und/oder formschlüssige Verbindung lösbar mit dem Gehäuse (1) der Injektionsvorrichtung verbunden sein.

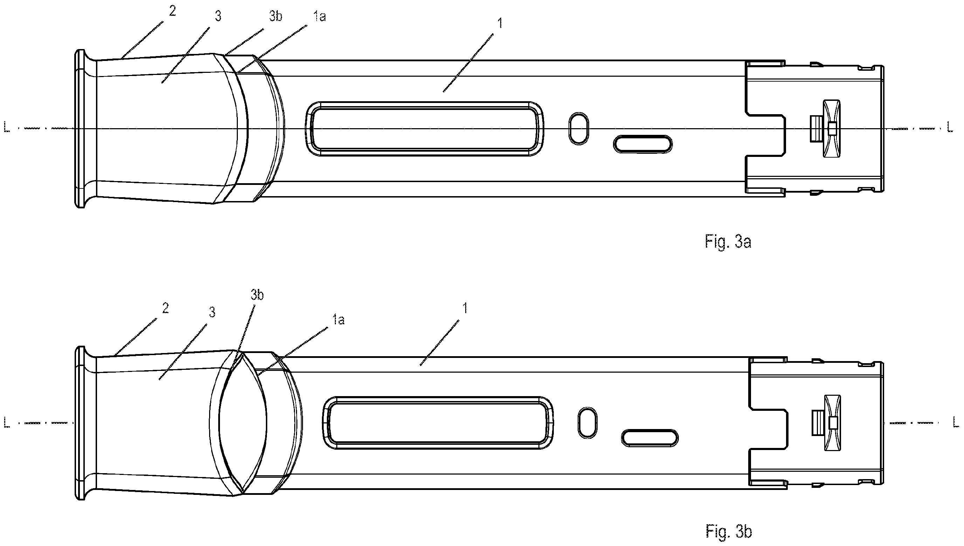

In der Figur 3a ist eine Aussenansicht der Injektionsvorrichtung gemäss Figur 2 ersichtlich. Die Figur 2 stellt die Injektionsvorrichtung in dem Auslieferungszustand dar. Der Benutzer dreht die Drehhülse (3) in eine Richtung, insbesondere in die Richtung, welche auf der Drehhülse (3) in Form eines Zeichens, insbesondere in Form eines Pfeils (nicht gezeigt) angezeigt ist, um die Kappe (2) von der Injektionsvorrichtung zu lösen. Dabei wird die lösbare Verbindung zwischen der Nadelschutzhülse (6) und der Entfernerhülse (5) gelöst, nämlich zwischen der Abragung (6a) der Nadelschutzhülse (6) und der Öffnung (5b) der Entfernerhülse (5). Ferner kann bei Drehung der Drehhülse (3) um die Längsachse (L) die reib- und/ oder formschlüssige Verbindung zwischen der Drehhülse (3) und dem Gehäuse (1) der Injektionsvorrichtung gelöst werden. Zudem gleitet der Federarm der Drehhülse (3) über die Rille, besonders bevorzugt über die Rillen, insbesondere über die Längsrillen, um eine Drehung der Drehhülse (3) in eine Richtung zu ermöglichen und in Gegenrichtung zu sperren. Alternativ gleitet die Rückdrehnocke (3c) der Drehhülse (3) über den Rückdrehvorsprung (5e) der Entfernerhülse (5), um eine Drehung der Drehhülse (3) um die Längsachse (L) in eine Richtung zu ermöglichen und in die Gegenrichtung zu sperren. Aufgrund der axial festen Verbindung zwischen der Drehhülse (3) und dem Eingriffselement (4) und der wellenförmigen oder kurvenförmigen Führungskulissen (3b; 1b) zwischen der Drehhülse (3) und dem Gehäuse (1) bewegt sich das Eingriffselement (4) gleichzeitig in die distale Richtung. Bei einer Drehung der Drehhülse um die Längsachse (L) um etwa 90° hat, wird die Drehhülse (3) und das Eingriffselement (4) relativ zu dem Gehäuse um einen axialen Weg in distaler Richtung bewegt, wie dies in der Figur 3b dargestellt ist. Bei der Dreh- und Axialbewegung der Drehhülse (3) und bei der Axialbewegung des Eingriffselements (4) in die distale Richtung gelangt oder ist das Eingriffselement (4) in Eingriffsposition mit der Nadelschutzkappe, um das Entfernen der Nadelschutzkappe von dem Produktbehälter (nicht gezeigt) zu bewirken. Nachdem der Benutzer durch Drehung der Drehhülse (3) um die Längsachse (L) die distale Position erreicht hat, kann der Benutzer die Kappe (2) vollständig von dem Injektionsgerät, insbesondere durch eine axiale Bewegung in distaler Richtung abnehmen, um danach mit der Injektionsvorrichtung eine Injektion zu tätigen. In einer alternativen Ausführungsform gleitet die Rückdrehnocke (3c) der Drehhülse (3) über den Rückdrehvorsprung (5e) der Entfernerhülse (5) bei Drehung der Drehhülse (3) um die Längsachse (L), wenn die Drehhülse die distalste Position erreicht hat oder kurz bevor die Drehhülse die distalste Position erreicht hat

### Bezugszeichen:

- 1: Gehäuse
- 1a: korrespondierende wellenförmige oder kurvenförmige Führungskulisse
- 2: Kappe
- 3: Drehhülse
- 3a: Nocke
- 3b: wellenförmige oder kurvenförmige Führungskulisse
- 3c: Rückdrehnocke
- 4: Eingriffselement
- 4a: Aussparung
- 5: Entfernerhülse
- 5a: Vorsprung
- 5b: Öffnung
- 5c: Ringnut
- 5d: Längsnut
- 5e: Rückdrehvorsprung
- 6: Nadelschutzhülse
- 6a: Abragung
- L: Längsachse

## Patentansprüche

1. Injektionsvorrichtung mit einer Längsachse (L) mit:
- einem Gehäuse (1) zur Aufnahme eines Produktbehälters, wobei der Produktbehälter eine fest verbundene Injektionsnadel aufweist, wobei an dem Produktbehälter eine Nadelschutzkappe lösbar angeordnet ist, welche die Injektionsnadel umschliesst und gegenüber der Umgebung steril abdichtet,
- eine Kappe (2), welche lösbar an einem distalen Ende des Gehäuses (1) vorgesehen ist,
- wobei die Kappe (2) ein Eingriffselement (4) umfasst, um beim Entfernen der Kappe (2) von der Injektionsvorrichtung das Entfernen der Nadelschutzkappe von dem Produktbehälter zu bewirken,
- wobei das Eingriffselement (4) relativ zu dem Gehäuse (1) drehfest angeordnet ist,
- wobei die Kappe eine Drehhülse (3) umfasst, wobei die Drehhülse (3) zumindest teilweise das Eingriffselement (4) aufgenommen hat, und wobei die Drehhülse (3) relativ zu dem Gehäuse (1) und relativ zu dem Eingriffselement (4) um die Längsachse (L) drehbar ist, und wobei die Drehhülse (3) relativ zu dem Eingriffselement (4) axialfest angeordnet ist,
- wobei die Drehhülse (3) und das Gehäuse (1) jeweils eine wellenförmige oder kurvenförmige Führungskulisse (3b; 1a) aufweisen, welche derart ausgebildet sind und derart zusammenwirken, dass die Drehhülse (3) relativ zu dem Gehäuse (1) um die Längsachse (L) drehbar ist und die Drehhülse (3) und das Eingriffselement (4) relativ zu dem Gehäuse (1) axial in die distale Richtung bewegbar ist,
**dadurch gekennzeichnet, dass**
- die Drehhülse (3) ein Rückdrehsicherungselement und das Eingriffselement (4) oder das Gehäuse (1) ein Rückdrehsicherungsgegenelement aufweist, um eine Drehung der Drehhülse (3) in eine Richtung zu ermöglichen und in Gegenrichtung zu sperren.

2. Injektionsvorrichtung nach Anspruch 1, **dadurch gekennzeichnet, dass** das Rückdrehsicherungselement der Drehhülse (3) und/oder das Rückdrehsicherungsgegenelement des Eingriffselements (4) oder des Gehäuses (1) eine Verzahnung oder Verrastung aufweist oder als Verzahnung oder Verrastung ausgebildet sind.

3. Injektionsvorrichtung nach Anspruch 1, **dadurch gekennzeichnet, dass** das Rückdrehsicherungselement der Drehhülse (3) und/oder das Rückdrehsicherungsgegenelement des Eingriffselements (4) oder des Gehäuses (1) einen Federarm aufweist oder als Federarm ausgebildet ist.

4. Injektionsvorrichtung nach einem der vorhergehenden Ansprüche, **dadurch gekennzeichnet, dass** die Drehhülse (3), das Eingriffselement (4) und/oder das Gehäuse (1) eine visuelle Markierung, insbesondere in Form eines Zeichens aufweist, um anzuzeigen in welche Richtung eine Drehung der Drehhülse (3) möglich ist.

5. Injektionsvorrichtung nach einem der vorhergehenden Ansprüche, **dadurch gekennzeichnet, dass** die Kappe ferner eine Entfernerhülse (5) aufweist, welche axialfest und drehfest mit dem Eingriffselement (4) verbunden ist und wobei die Drehhülse (3) relativ zu der Entfernerhülse drehbar angeordnet ist.

6. Injektionsvorrichtung nach Anspruch 5, **dadurch gekennzeichnet, dass** die Drehhülse (3) und die Entfernerhülse (5) in einem Führungseingriff (4) sind, derart, dass die Drehhülse (3) relativ zu der Entfernerhülse (5) um die Längsachse (L) drehbar ist.

7. Injektionsvorrichtung nach einem der vorhergehenden Ansprüche, **dadurch gekennzeichnet, dass** das Eingriffselement (4) einen oder mehrere Haken aufweist oder hakenförmig ausgebildet ist.

8. Injektionsvorrichtung nach einem der vorhergehenden Ansprüche, **dadurch gekennzeichnet, dass** das Eingriffselement (4) und die Drehhülse (3) zueinander konzentrisch angeordnet sind.

9. Injektionsvorrichtung nach einem der vorhergehenden Ansprüche, **dadurch gekennzeichnet, dass** ferner eine Nadelschutzhülse (6) vorgesehen ist, wobei die Kappe (2) über die Nadelschutzhülse (6) mit dem Gehäuse (1) lösbar verbunden ist.

10. Verfahren zum Vorbereiten einer Injektionsvorrichtung für die Verabreichung eines Produkts mit folgenden Schritten:
- Bereitstellen einer Injektionsvorrichtung nach einem der vorhergehenden Ansprüche,
- Drehung der Drehhülse (3) um die Längsachse (L) und gleichzeitige axiale Bewegung des Eingriffselements (4) in die distale Richtung, wobei das Eingriffselement (4) in Eingriff mit der Nadelschutzkappe ist oder gebracht wird.

## Claims

1. Injection device having a longitudinal axis (L), comprising:
- a housing (1) for receiving a product container, the product container having a fixedly connected injection needle, a needle protection cap being detachably arranged on the product container, which needle protection cap encloses the injection needle and seals the injection needle in a sterile manner,
- a cap (2), which is detachably provided at a distal end of the housing (1),
- the cap (2) comprising an engagement element (4) in order to cause the needle protection cap to be removed from the product container when the cap (2) is removed from the injection device,
- the engagement element (4) being arranged in a rotationally fixed manner relative to the housing (1),
- the cap comprising a rotary sleeve (3), the rotary sleeve (3) having received the engagement element (4) at least in part, and the rotary sleeve (3) being rotatable relative to the housing (1) and relative to the engagement element (4) about the longitudinal axis (L), and the rotary sleeve (3) being arranged in an axially fixed manner relative to the engagement element (4),
- the rotary sleeve (3) and the housing (1) each having an undulating or curved guide slot (3b; 1a) which are designed and interact in such a way that the rotary sleeve (3) is rotatable relative to the housing (1) about the longitudinal axis (L) and the rotary sleeve (3) and the engagement element (4) can be moved axially in the distal direction relative to the housing (1),
**characterized in that**
- the rotary sleeve (3) has an anti-reverse-rotation element and the engagement element (4) or the housing (1) has an anti-reverse-rotation counter element in order to allow a rotation of the rotary sleeve (3) in one direction and to block a rotation of the rotary sleeve in the opposite direction.

2. Injection device according to claim 1, **characterized in that** the anti-reverse-rotation element of the rotary sleeve (3) and/or the anti-reverse-rotation counter element of the engagement element (4) or of the housing (1) has a toothing or latching or are designed as a toothing or latching.

3. Injection device according to claim 1, **characterized in that** the anti-reverse-rotation element of the rotary sleeve (3) and/or the anti-reverse-rotation counter element of the engagement element (4) or of the housing (1) has a spring arm or is designed as a spring arm.

4. Injection device according to any of the preceding claims, **characterized in that** the rotary sleeve (3), the engagement element (4) and/or the housing (1) has a visual marking, in particular in the form of a symbol, in order to indicate in which direction a rotation of the rotary sleeve (3) is possible.

5. Injection device according to any of the preceding claims, **characterized in that** the cap further comprises a remover sleeve (5) which is connected to the engagement element (4) in an axially fixed and rotationally fixed manner and the rotary sleeve (3) being arranged so as to be rotatable relative to the remover sleeve.

6. Injection device according to claim 5, **characterized in that** the rotary sleeve (3) and the remover sleeve (5) are in a guide engagement (4) such that the rotary sleeve (3) is rotatable relative to the remover sleeve (5) about the longitudinal axis (L).

7. Injection device according to any of the preceding claims,
**characterized in that** the engagement element (4) has one or more hooks or is hook-shaped.

8. Injection device according to any of the preceding claims,
**characterized in that** the engagement element (4) and the rotary sleeve (3) are arranged concentrically to one another.

9. Injection device according to any of the preceding claims,
**characterized in that** a needle protection sleeve (6) is also provided, the cap (2) being detachably connected to the housing (1) via the needle protection sleeve (6).

10. Method for preparing an injection device for administering a product, comprising the steps of:
- providing an injection device according to any of the preceding claims,
- rotating the rotary sleeve (3) about the longitudinal axis (L) and simultaneously axially moving the engagement element (4) in the distal direction, wherein the engagement element (4) is in engagement with or is brought into engagement with the needle protection cap.

## Revendications

1. Dispositif d'injection comportant un axe longitudinal (L) comportant :
- un boîtier (1) permettant de recevoir un récipient de produit, le récipient de produit présentant une aiguille d'injection reliée de manière fixe, un capuchon de protection d'aiguille étant disposé de manière amovible sur le récipient de produit, lequel capuchon entoure l'aiguille d'injection et réalise l'étanchéité de celle-ci de manière stérile vis-à-vis de l'environnement,
- un capuchon (2), lequel est prévu de manière amovible au niveau d'une extrémité distale du boîtier (1),
- le capuchon (2) comprenant un élément de prise (4) pour provoquer, lors de l'enlèvement du capuchon (2) du dispositif d'injection, l'enlèvement du capuchon de protection d'aiguille du récipient de produit,
- l'élément de prise (4) étant disposé de manière solidaire en rotation par rapport au boîtier (1),
- le capuchon comprenant un manchon rotatif (3), le manchon rotatif (3) ayant reçu au moins partiellement l'élément de prise (4), et le manchon rotatif (3) pouvant tourner par rapport au boîtier (1) et par rapport à l'élément de prise (4) autour de l'axe longitudinal (L), et le manchon rotatif (3) étant disposé de manière axialement fixe par rapport à l'élément de prise (4),
- le manchon rotatif (3) et le boîtier (1) présentant respectivement une coulisse de guidage (3b ; 1a) ondulée ou curviligne, lesquelles sont réalisées et coopèrent de telle sorte que le manchon rotatif (3) peut tourner par rapport au boîtier (1) autour de l'axe longitudinal (L) et le manchon rotatif (3) et l'élément de prise (4) peuvent être déplacés par rapport au boîtier (1) axialement dans la direction distale,
**caractérisé en ce que**
- le manchon rotatif (3) présente un élément de sécurité anti-rotation en sens inverse et l'élément de prise (4) ou le boîtier (1) présente un contre-élément de sécurité anti-rotation en sens inverse pour permettre une rotation du manchon rotatif (3) dans un sens et pour la bloquer dans le sens contraire.

2. Dispositif d'injection selon la revendication 1, **caractérisé en ce que** l'élément de sécurité anti-rotation en sens inverse du manchon rotatif (3) et/ou le contre-élément de sécurité anti-rotation en sens inverse de l'élément de prise (4) ou du boîtier (1) présentent une denture ou un enclenchement ou sont réalisés sous forme de denture ou d'enclenchement.

3. Dispositif d'injection selon la revendication 1, **caractérisé en ce que** l'élément de sécurité anti-rotation en sens inverse du manchon rotatif (3) et/ou le contre-élément de sécurité anti-rotation en sens inverse de l'élément de prise (4) ou du boîtier (1) présentent un bras de ressort ou sont réalisés sous forme de bras de ressort.

4. Dispositif d'injection selon l'une quelconque des revendications précédentes, **caractérisé en ce que** le manchon rotatif (3), l'élément de prise (4) et/ou le boîtier (1) présentent un repère visuel, en particulier sous la forme d'un signe, pour indiquer dans quel sens une rotation du manchon rotatif (3) est possible.

5. Dispositif d'injection selon l'une quelconque des revendications précédentes, **caractérisé en ce que** le capuchon présente en outre un manchon d'enlèvement (5), lequel est relié à l'élément de prise (4) de manière axialement fixe et solidaire en rotation et le manchon rotatif (3) étant disposé de manière à pouvoir tourner par rapport au manchon d'enlèvement.

6. Dispositif d'injection selon la revendication 5, **caractérisé en ce que** le manchon rotatif (3) et le manchon d'enlèvement (5) sont en prise de guidage (4) de telle sorte que le manchon rotatif (3) peut tourner par rapport au manchon d'enlèvement (5) autour de l'axe longitudinal (L).

7. Dispositif d'injection selon l'une quelconque des revendications précédentes, **caractérisé en ce que** l'élément de prise (4) présente un ou plusieurs crochets ou est réalisé en forme de crochet.

8. Dispositif d'injection selon l'une quelconque des revendications précédentes, **caractérisé en ce que** l'élément de prise (4) et le manchon rotatif (3) sont disposés de manière concentrique l'un par rapport à l'autre.

9. Dispositif d'injection selon l'une quelconque des revendications précédentes, **caractérisé en ce qu'**un manchon de protection d'aiguille (6) est en outre prévu, le capuchon (2) étant relié de manière amovible au boîtier (1) par l'intermédiaire du manchon de protection d'aiguille (6).

10. Procédé permettant de préparer un dispositif d'injection pour l'administration d'un produit comportant les étapes suivantes :
- fourniture d'un dispositif d'injection selon l'une quelconque des revendications précédentes,
- rotation du manchon rotatif (3) autour de l'axe longitudinal (L) et déplacement axial simultané de l'élément de prise (4) dans la direction distale, l'élément de prise (4) étant ou étant amené en prise avec le capuchon de protection d'aiguille.
